(19) Europäisches Patentamt / European Patent Office / Office européen des brevets

(11) **EP 2 719 287 B1**

(12) **EUROPEAN PATENT SPECIFICATION**

(45) Date of publication and mention of the grant of the patent:
**04.12.2019 Bulletin 2019/49**

(21) Application number: **13187870.4**

(22) Date of filing: **09.10.2013**

(51) Int Cl.:
*A61K 36/752* (2006.01)   *A61K 31/045* (2006.01)
*A61K 31/366* (2006.01)   *A61K 31/7048* (2006.01)
*A61P 3/06* (2006.01)   *A61P 17/18* (2006.01)
*A61Q 17/00* (2006.01)   *A61K 31/122* (2006.01)
*A61K 8/00* (2006.01)   *A61Q 17/04* (2006.01)
*A61P 17/16* (2006.01)   *A61K 8/34* (2006.01)
*A61K 8/49* (2006.01)   *A61K 8/60* (2006.01)
*A23L 33/10* (2016.01)   *A61K 36/899* (2006.01)
*A61Q 19/00* (2006.01)   *A23L 33/105* (2016.01)

(54) **Compositions for the treatment of hyperlipidemia**

Zusammensetzungen zur Behandlung der Hyperlipämie

Compositions pour le traitement de l'hyperlipidémie

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2012 IT MI20121696**

(43) Date of publication of application:
**16.04.2014 Bulletin 2014/16**

(73) Proprietors:
• **DDFARMA S.R.L.**
  **20900 Monza (MB) (IT)**
• **AURORA BIOFARMA S.R.L.**
  **20131 Milano (IT)**

(72) Inventor: **Damiani, Luciano Marco**
**20900 MONZA (IT)**

(74) Representative: **Coppo, Alessandro et al**
Notarbartolo & Gervasi S.p.A.
Corso di Porta Vittoria, 9
20122 Milano (IT)

(56) References cited:
WO-A1-99/25316       WO-A1-03/030838
WO-A1-2006/037725    WO-A1-2007/113748

• anonymous: "No-colest omegasol", internet , 2011, XP002698228, Retrieved from the Internet: URL:http://www.lerborista.it/Integratori-E rboristici/Colesterolo-e-trigliceridi/No-C olest-Omegasol-30-perle-in-blister/#.Ua33l qz4J7k [retrieved on 2013-06-05]

• Giuseppe Maria Ricchiuto: "Prenditi cura del tuo cuore (salotto benessere)", internet Viaggio in benessere, no. 4 September 2011 (2011-09), pages 88-89, XP002698227, Italy Retrieved from the Internet: URL:http://en.calameo.com/read/0008751769a c4cc724560 [retrieved on 2013-06-05]

• MOLLACE V ET AL: "Hypolipemic and hypoglycaemic activity of bergamot polyphenols: From animal models to human studies", FITOTERAPIA 2011 ELSEVIER NLD, vol. 82, no. 3, April 2011 (2011-04), pages 309-316, XP002698316, ISSN: 0367-326X

• GOUNI-BERTHOLD I ET AL: "Policosanol: Clinical pharmacology and therapeutic significance of a new lipid-lowering agent", AMERICAN HEART JOURNAL, MOSBY- YEAR BOOK INC, US, vol. 143, no. 2, 1 February 2002 (2002-02-01), pages 356-365, XP002263555, ISSN: 0002-8703, DOI: 10.1067/MHJ.2002.119997

• Anonymous: "Policosanol: Martindale: The Complete Drug Reference", , 8 December 2017 (2017-12-08), XP055433677, Retrieved from the Internet: URL:https://www.medicinescomplete.com/mc/m artindale/2009/ms-17176-a.htm#m17176-a [retrieved on 2017-12-08]

Remarks:
The file contains technical information submitted after the application was filed and not included in this specification

**Description**

**FIELD OF THE INVENTION**

The present invention relates to a composition comprising a mixture of neoeriocitrin, naringin and neohesperidin, in association with monacolin K and policosanols. In particular, such a composition showed a surprising synergistic effect such as to make it suitable for the preparation of food supplements and pharmaceutical formulations.

**STATE OF THE ART**

The term 'hyperlipidemia' indicates any alteration of the lipid metabolism which results in an increase in some or all of the types of lipids and/or lipoproteins in the blood. The alteration may appear as hypertriglyceridemia, hypercholesterolemia, or both.

Hypercholesterolemia means an excess of cholesterol in the blood; more specifically, it refers to an increase of cholesterol carried by low density lipoproteins (LDL), commonly known as "bad cholesterol".

Cholesterol, as all lipids, is not water-soluble, thus it is carried by proteins in the blood, which are called apolipoproteins (APO). The complex formed by apolipoproteins, cholesterol, triglycerides and phospholipids constitutes lipoproteins, relatively large particles that circulate in the blood for the purpose of carrying fats towards all tissues.

Under fasting conditions (i.e. under blood test conditions), the cholesterol present in the blood is for the most part (60-75%) the one carried by LDL, so the dosage of the total plasma cholesterol is an index, although approximate, of LDL cholesterol. However, since a good percentage of cholesterol is carried by other lipoproteins (VLDL and HDL), a determination of LDLs is preferable for a more exact evaluation of cholesterolemia. This mode allows a distinction between LDL cholesterol ("bad" cholesterol) and HDL cholesterol ("good" cholesterol).

The LDLs (which are a product of the hepatic synthesis metabolism of VLDLs) carry cholesterol from the liver to the tissues, where it is used for a variety of processes; however, when the LDLs are present in high concentrations, their accumulation in the arterial wall promotes the development of atherosclerosis. Consequently, LDL hypercholesterolemia is one of the major risk factors for cardiovascular diseases.

In contrast, HDLs are responsible for the "reverse transport" of cholesterol, i.e. they remove excess cholesterol from the tissues and carry it to the liver. Hence, it is eliminated in the intestinal lumen partly as bile salts and partly as free cholesterol. HDLs therefore play a protection role on the development of cardiovascular diseases. An excess of HDL cholesterol is therefore a favorable facto r.

The identification of hypercholesterolemia as a cardiovascular risk factor (i.e., a factor that increases the likelihood of developing a cardiovascular event) is the result of a long series of epidemiological studies that have shown the correlation between cholesterolemia (cholesterol values in plasma) and cardiovascular ischemic events, primarily myocardial infarction and cardiovascular mortality, of which myocardial infarction and stroke are the most common causes.

The international patent application WO 2006/037725A1 discloses the use of compositions comprising red yeast rice, policosanols and coenzyme Q 10 for the treatment of hyperlipidemia.

The need for a product that can effectively reduce the levels of blood lipids, and in particular the levels of cholesterol, is therefore felt, which product should be both highly biocompatible and tolerable by the body.

**SUMMARY OF THE INVENTION**

Such an object is achieved by a composition comprising monacolin K, policosanols and a mixture of neoeriocitrin, naringin and neohesperidin, wherein said monacolin K and said mixture of neoeriocitrin, naringin and neohesperidin are in a weight ratio from 1:5 to 1:20 and said policosanols are in the form of a mixture comprising at least 98% w/w C24-C40 fatty alcohols, of which at least 60% w/w 1-octacosanol.

In another aspect, the present invention relates to a dietary supplement comprising said composition.

In another aspect, the present invention relates to a pharmaceutical formulation comprising said composition.

In another aspect, the present invention relates to the use of said composition as an antioxidant agent in the treatment of hyperlipidemia.

In another aspect, the present invention relates to the use of said composition as an antioxidant agent in the treatment of UV radiation skin protection.

In a further aspect, the present invention relates to the use of said dietary supplement or said pharmaceutical composition in the treatment of hyperlipidemia. In another aspect, the present invention relates to the use of said dietary supplement or said pharmaceutical composition in the treatment of UV radiation skin protection.

**DESCRIPTION OF THE FIGURE**

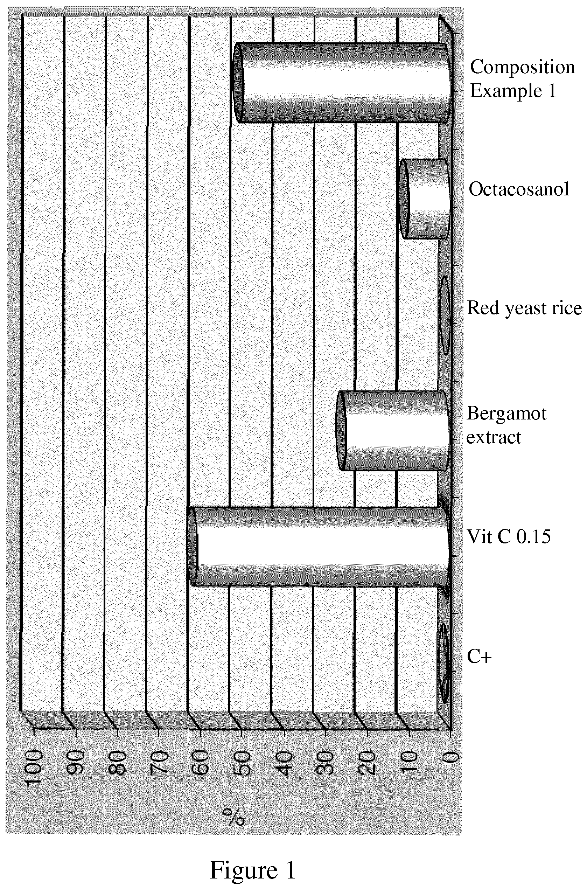

The features and the advantages of the present invention will be clear from the following detailed description, from the embodiments provided as, illustrative examples, as well as from Figure 1 which shows the outcome of the evaluation of the antioxidant activity of the composition of the invention, as per Example 8.

**DETAILED DESCRIPTION OF THE INVENTION**

The present invention therefore relates to a composition comprising monacolin K, policosanols and a mixture of neoeriocitrin, naringin and neohesperidin, wherein said monacolin K and said mixture of neoeriocitrin, naringin and neohesperidin are in a weight ratio from 1:5 to 1:20 and said policosanols are in the form of a mixture comprising at least 98% w/w C24-C40 fatty alcohols, of which at least 60% w/w 1-octacosanol. In fact, it has been observed that the combination of such substances has surprising synergistic antioxidant properties so as to lower the LDLs.
Where not otherwise specified, all the ratios of the present invention are expressed by weight.
Preferably, said monacolin K and said mixture of neoeriocitrin, naringin and neohesperidin are in a weight ratio from 1:10 to 1:18, more preferably from 1:12 to 1:15, more preferably from 1:12.5 to 1:14.
In some embodiments, said policosanols and said mixture of neoeriocitrin, naringin and neohesperidin are in a weight ratio from 1:3 to 1:10, preferably from 1:6 to 1:7. In other embodiments, said policosanols and said mixture of neoeriocitrin, naringin and neohesperidin are in a weight ratio from 5:1 to 1:2, preferably from 3:1 to 1:1.
According to a preferred embodiment, said mixture of neoeriocitrin, naringin and neohesperidin is in the form of a Bergamot extract (*Citrus aurantium Bergamia* fruit extract). The analysis of said extract in order to evaluate the content of the mixture for the purposes of the present invention can be conducted by HPLC. Preferably, said mixture of neoeriocitrin, naringin and neohesperidin is a Bergamot extract comprising 20-50% w/w neoeriocitrin, naringin and neohesperidin, more preferably 25-30% w/w neoeriocitrin, naringin and neohesperidin.
In other embodiments, the composition of the invention consists of monacolin K, policosanols in the form of a mixture comprising at least 98% w/w C24-C40 fatty alcohols, of which at least 60% w/w 1-octacosanol and a mixture of neoeriocitrin, naringin and neohesperidin, wherein said monacolin K and said mixture of neoeriocitrin, naringin and neohesperidin are in a weight ratio from 1:5 to 1:20. For purposes of the present invention, both said mixture of neoeriocitrin, naringin and neohesperidin and said Bergamot extract are free from bergamottin, a toxic and photo-mutagenic substance that is present in the peel of Bergamot fruits. According to another embodiment, said monacolin K is in the form of powder of red yeast rice titrated in monacolin K; more specifically, it is red yeast rice from *Monascus purpureus.* Preferably, said monacolin K is in the form of powder of red yeast rice titrated to 1 to 5% monacolin K.
Preferably, said C24-C40 fatty alcohols, are 1-tetracosanol, 1-hexacosanol, 1-octacosanol, 1-triacotanol, 1-entriacotanol, and 1-nonacosanol.
According to other embodiments, the composition of the invention further comprises coenzyme Q10.
In other embodiments, the composition of the invention consists of a mixture of neoeriocitrin, naringin and neohesperidin, monacolin K, policosanols and coenzyme Q10, wherein said monacolin K and said mixture of neoeriocitrin, naringin and neohesperidin are in a weight ratio from 1:5 to 1:20 and said policosanols are in the form of a mixture comprising at least 98% w/w C24-C40 fatty alcohols, of which at least 60% w/w 1-octacosanol.
In other embodiments, the only active ingredients present in the composition of the invention are the mixture of neoeriocitrin, naringin and neohesperidin, monacolin K, policosanols and coenzyme Q10, wherein said monacolin K and said mixture of neoeriocitrin, naringin and neohesperidin are in a weight ratio from 1:5 to 1:20 and said policosanols are in the form of a mixture comprising at least 98% w/w C24-C40 fatty alcohols, of which at least 60% w/w 1-octacosanol.
Alternatively, the composition of the invention consists of monacolin K, policosanols, coenzyme Q10, mixture of neoeriocitrin, naringin and neohesperidin and suitable excipients, wherein said monacolin K and said mixture of neoeriocitrin, naringin and neohesperidin are in a weight ratio from 1:5 to 1:20 and said policosanols are in the form of a mixture comprising at least 98% w/w C24-C40 fatty alcohols, of which at least 60% w/w 1-octacosanol.
Preferably, said monacolin K and said coenzyme Q10 are in a weight ratio from 10:1 to 1:1, more preferably, from 7:1 to 2:1.
In other embodiments, the composition of the invention comprises 5-20% by weight of a mixture of neoeriocitrin, naringin and neohesperidin, 0.4-2% by weight of monacolin K, 0.5-2% by weight of policosanols as defined above, on total composition weight.
Preferably, said composition comprises 7-12% by weight of a mixture of neoeriocitrin, naringin and neohesperidin, 0.5-1% by weight of monacolin K, 0.8-1.7% by weight of policosanols as defined above, on total composition weight. More preferably, such a composition comprises 8-10% by weight of a mixture of neoeriocitrin, naringin and neohesperidin, 0.6-0.8% by weight of monacolin K, 1-1.5% by weight of policosanols as defined above, on total composition weight. Even more preferably, such a composition comprises 8.2-9.3% by weight of a mixture of neoeriocitrin, naringin and neohesperidin, 0.65-0.7% by weight of monacolin K, 1.3-1.4% by weight of policosanols as defined above, on total composition weight.

The composition of the invention may be in the form of a unit dose comprising 15-240 mg of said mixture of neoeriocitrin, naringin and neohesperidin, 2 to 10.5 mg of monacolin K, 3.5-25 mg of policosanols ad defined above.

Preferably, the composition is in the form of a unit dose comprising 50-150 mg of said mixture of neoeriocitrin, naringin and neohesperidin, 3.5 to 10.5 mg of monacolin K, 6-20 mg of policosanols as defined above.

More preferably, the composition is in the form of a unit dose comprising 61-76 mg of said mixture of neoeriocitrin, naringin and neohesperidin, 4.5 to 10.5 mg of monacolin K, 7.5-11.5 mg of policosanols as defined above.

When the mixture of neoeriocitrin, naringin and neohesperidin is in the form of a Bergamot extract, the unit dose preferably comprises 200-1000 mg of the Bergamot extract.

When the monacolin K is in the form of red yeast rice titrated in monacolin K, the unit dose preferably comprises 100-700 mg of red yeast rice.

When coenzyme Q10 is present, the unit dose comprises 1-10 mg of coenzyme Q10, preferably 1-6 mg of coenzyme Q10.

In another aspect, the present invention relates to a dietary supplement comprising the composition as described above and suitable food excipients. In particular, suitable excipients are natural starch, partially hydrolyzed starch, lactose, glucose, sucrose, mannitol, sorbitol, cellulose and derivatives thereof, microcrystalline cellulose and derivatives thereof, calcium phosphate, calcium carbonate, calcium sulfate, magnesium stearate, maltodextrin, gelatin, gum tragacanth, arabic gum, xanthan gum, talc, silica, colloidal silica, precipitated silica, magnesium silicates, aluminum silicates, sodium lauryl sulfate, magnesium lauryl sulfate, methacrylate copolymers, and mixtures thereof.

Said dietary supplement can be in the form of powder, capsule, tablet, mini-tablet, micro-tablet, granule, micro-granule, pellet, multi particulate, or micronized particulate. Alternatively, it may be in liquid form, i.e. in solution with suitable solvents.

According to a preferred embodiment, the dietary supplement comprises:

- 62-72 mg of mixture of neoeriocitrin, naringin and neohesperidin, in the form of a Bergamot extract,
- 4.8-5.5 mg of monacolin K in the form of red yeast rice titrated at 3% monacolin K,
- 9-11 mg of policosanols as defined above,
- 1-4 mg of coenzyme Q10, and
- 250-400 mg of food excipients.

The most preferred embodiment is the dietary supplement of Example 7 below, comprising:

- 250 mg of a Bergamot extract,
- 167 mg of red yeast rice trit. at 3% (Oryza sativa L.) seed trit. at 3% monacolin K,
- 10 mg of policosanols from sugar cane (*Saccharum officina rum L.*) as defined above, and
- 2 mg of Coenzyme Q10.

In a further aspect, the present invention relates to a pharmaceutical formulation comprising the composition, as described above, and pharmaceutically acceptable excipients. Suitable pharmaceutically acceptable excipients are diluents, disintegrants, glidants, binders, lubricants, stabilizers, adsorbents, release retardants and preservatives.

Such a pharmaceutical formulation can be in the form of powder, capsule, tablet, mini-tablet, micro-tablet, granule, micro-granule, pellet, multi particulate, or micronized particulate. Alternatively, it may be in liquid form, i.e. in solution with suitable solvents.

In another aspect, the present invention relates to the use of the composition, as described above, in the treatment of hyperlipidemia. In fact, a surprising synergistic antioxidant activity was observed, as will be seen in the following examples.

Similarly, also the dietary supplement and the pharmaceutical formulation comprising the composition can be employed in the treatment of hyperlipidemia.

In another aspect, the present invention relates to the use of the composition as an antioxidant agent in the treatment of UV radiation skin protection.

Similarly, also the dietary supplement and the pharmaceutical formulation comprising the composition can be employed in the treatment of UV radiation skin protection.

In fact, as will be seen from the following Examples, the composition of the invention showed surprising synergistic antioxidant properties on human keratinocytes, which allow the composition of the invention to effectively counteract skin aging, the onset of skin irritation and diseases of different importance.

Preferably, the composition of the invention is administered orally.

More preferably, said composition is administered in such an amount as to provide a daily dose containing from 5 to 10 mg of monacolin K.

The composition according to the present invention, as well as the dietary supplement and pharmaceutical formulation, can be prepared by methods known in the art. In fact, for oral administration, the components can, for example, be mixed as such or with one or more excipients, enclosed in gelatin capsules or compressed into tablets. Otherwise, for example in the case of parenteral administration, the liquid compositions can be prepared by mixing the components with suitable diluents and/or excipients.

It should be understood that all aspects identified as preferred and advantageous for the composition of the invention, are to be considered as similarly preferred and advantageous also for the dietary supplement, the pharmaceutical formulation and the relevant uses.

Below are Examples of preparation of the compounds according to the present invention, as well as examples of evaluation of their effectiveness, provided for illustrative purposes.

EXAMPLES

**Example 1.**

**Preparation of a composition according to the invention**

A composition as reported in Table 1 was prepared.

Table 1.

|  | Amount |
|---|---|
| Bergamot extract<br>wherein mixture of neoeriocitrin, naringin and neohesperidin (25-28%w/w) | 250 mg<br>(62,5-70 mg) |
| Red yeast rice<br>wherein monacolin K (3%) | 167 mg<br>(5 mg) |
| Polycosanols in the form of a mixture comprising at least 98% w/w C24-C40 fatty alcohols of which 60% octacosanol | 10 mg |
| Total | 427 mg |

**Example 2.**

**Preparation of a composition according to the invention**

A composition as reported in Table 2 was prepared.

Table 2.

|  | Amount |
|---|---|
| Bergamot extract<br>wherein mixture of neoeriocitrin, naringin and neohesperidin (25-28%w/w) | 500 mg<br>(125-140 mg) |
| Red yeast rice<br>wherein monacolin K (3%) | 334 mg<br>(10 mg) |
| Polycosanols in the form of a mixture comprising at least 98% w/w C24-C40 fatty alcohols, of which at least 60% w/w 1-octacosanol | 20 mg |
| Excipients(*) | 146 mg |
| Total | 1000 mg |
| (*) maltodextrin, silica, magnesium stearate | |

**Example 3.**

**Preparation of a composition according to the invention**

A composition as reported in Table 3 was prepared.

Table 3.

| | Amount |
|---|---|
| Bergamot extract<br>wherein mixture of neoeriocitrin, naringin and neohesperidin (25-28%w/w) | 500 mg<br>(125-140 mg) |
| Red yeast rice<br>wherein monacolin K (3%) | 334 mg<br>(10 mg) |
| Polycosanols in the form of a mixture comprising at least 98% w/w C24-C40 fatty alcohols, of which at least 60% w/w 1-octacosanol | 20 mg |
| Coenzyme Q10 | 4 mg |
| Excipients(*) | 146 mg |
| Total | 1004 mg |
| (*) microcrystalline cellulose, maltodextrin | |

**Example 4.**

**Preparation of a composition according to the invention**

A composition as reported in Table 4 was prepared.

Table 4.

| | Amount |
|---|---|
| Bergamot extract<br>wherein mixture of neoeriocitrin, naringin and neohesperidin (25-28%w/w) | 200 mg<br>(50-56 mg) |
| Red yeast rice<br>wherein monacolin K (3%) | 167 mg<br>(5 mg) |
| Polycosanols in the form of a mixture comprising at least 98% w/w C24-C40 fatty alcohols, of which at least 60% w/w 1-octacosanol | 10 mg |
| Coenzyme Q10 | 2 mg |
| Excipients(*) | 421 mg |
| Total | 800 mg |
| (*) sorbitol, maltodextrin, magnesium stearate | |

**Example 5.**

**Preparation of a composition according to the invention**

A composition as reported in Table 5 was prepared.

Table 5.

| | Amount |
|---|---|
| Bergamot extract<br>wherein mixture of neoeriocitrin, naringin and neohesperidin (25-28%w/w) | 300 mg<br>(75-84 mg) |

(continued)

| | Amount |
|---|---|
| Red yeast rice<br>wherein monacolin K (3%) | 150 mg<br>(4,5 mg) |
| Polycosanols in the form of a mixture comprising at least 98% w/w C24-C40 fatty alcohols, of which at least 60% w/w 1-octacosanol | 8 mg |
| Excipients(*) | 342 mg |
| Total | 800 mg |
| (*) maltodextrin, silica, magnesium stearate | |

**Example 6.**

**Preparation of a composition according to the invention**

A composition as reported in Table 6 was prepared.

Table 6.

| | Amount |
|---|---|
| Bergamot extract<br>wherein mixture of neoeriocitrin, naringin and neohesperidin (25-28%w/w) | 480 mg<br>(120-134,4 mg) |
| Red yeast rice<br>wherein monacolin K (3%) | 334 mg<br>(10 mg) |
| Polycosanols in the form of a mixture comprising at least 98% w/w C24-C40 fatty alcohols, of which at least 60% w/w 1-octacosanol | 15 mg |
| Coenzyme Q10 | 6 mg |
| Total | 835 mg |

**Example 7.**

**Preparation of a composition according to the invention**

A composition as reported in Table 7 was prepared.

Table 7.

| | Amount |
|---|---|
| Bergamot extract<br>wherein mixture of neoeriocitrin, naringin and neohesperidin (25-28%w/w) | 250 mg<br>(62,5-70 mg) |
| Red yeast rice<br>wherein monacolin K (3%) | 167 mg<br>(5 mg) |
| Polycosanols in the form of a mixture comprising at least 98% w/w C24-C40 fatty alcohols, of which at least 60% w/w 1-octacosanol | 10 mg |
| Coenzyme Q10 | 2 mg |
| Excipients(*) | 331 mg |
| Total | 760 mg |
| (*) microcrystalline cellulose, sorbitol, maltodextrin, silica, magnesium stearate | |

**Example 8.**

***In vitro* comparative evaluation of the anti-oxidant activity of active ingredients through the investigation of their anti-scavenger activity on human keratinocyte cell cultures**

In order to evaluate whether the substances under examination possess the activity to neutralise reactive oxygen species (ROS), an *in vitro* measurement of the amount of ROS produced by cells after an induced oxidative stress, with respect to un-treated controls.

The following samples were prepared:

- positive Control (C+): un-treated cells exposed to UV, and cells exposed to UV in the presence of:
- Vitamin C 0.15 mg/ml (anti-oxidant of reference),
- Bergamot extract (25-28% w/w neoeriocitrin, naringin and neohesperidin),
- Red yeast rice (3% monacolin K),
- 1-Octacosanol,
- Composition of Example 1.

The single substances as well as the composition of Example 1 were diluted at a concentration of 0.6 mg/ml. The dilutions to the final desired concentrations were performed by adding a saline solution.

In the tests, appropriate controls were adopted and Vitamin C 0.15 mg/ml was used as a known anti-oxidant of reference.

Primary cell cultures of keratinocytes were used deriving from human epidermis biopsies. For the experiments, the cells were homogeneously seeded in 96 wells' plates.

Separately, dichlorofluorescein acetate (DCA) was solubilised in a suitable buffer. DCA is known to react with free radicals, if present, and form a fluorescent derivative. The fluorimetry reading allows to attain a quantitative datum, which is related to the ROS concentration in the cells. An appropriate number of cells (20,000 cells/well, 24° passage) was seminated in a 96 wells' plate. After an overnight pre-incubation with samples at different concentrations, the culture medium is dried out from the plates and replaced with 500 $\mu$l of DCA solution. Plates are incubated at 37°C for 15' in a $CO_2$ thermostat, then DCA solution is discharged. In order to induce an oxidative stress, the plate is exposed to UV for 4-8-12-16-20 minutes. After UV exposure, fluorescence is checked. The lamp used in the experiments is a solar light simulator with a constant emission in the UVA range (315-400 nm). The UVB emission was appropriately screened in order to avoid cytotoxic damages to cell cultures. Cells were irradiated at room temperature, with an intensity of 1.7 $mW/cm^2$ of UVA (= 5 $J/cm^2$).

The fluorimeter reading was made at an excitation wavelenght of 485 nm, and at an emission wavelenght of 530 nm, directly on the plates.

*Results*

After exposure to UV, the results were given in terms of fluorescence, which is proportional to the ROS amount:

| C+ | VIT C | Bergamot extract | red yeast rice | 1-octacosanol | Comp. Ex. 1 |
|---|---|---|---|---|---|
| 139229.9 | 55053.8 | 104616.0 | 138427.75 | 125356.5 | 69867.60 |

The percentage of anti-ROS protection showed by the samples was calculated through the following formula:

$$\% \ protection = [(ROS \ (positive \ Control) - ROS \ (Sample))/(ROS \ (positive \ Control))]*100$$

Said percentages are reported below, as well as are represented in Figure 1:

| C+ | VIT C | Bergamot extract | red yeast rice | 1-octacosanol | Comp. Ex. 1 |
|---|---|---|---|---|---|
| - | 60.46 | 24.86 | - | 9.96 | 49.82 |

It was thus surprisingly observed that the composition of the invention showed an antioxidant activity of 30% higher than the sum of the single substances' contribution, where it should be noted that red yeast rice shows no activity at all. The composition of the invention therefore demonstrated an unexpected synergistic effect with respect to the sum of the separate administrations of its components.

**Claims**

1. Composition comprising monacolin K, policosanols and a mixture of neoeriocitrin, naringin and neohesperidin, wherein said monacolin K and said mixture of neoeriocitrin, naringin and neohesperidin are in a weight ratio of 1:5 to 1:20 and said policosanols are in the form of a mixture comprising at least 98% w/w C24-C40 fatty alcohols, of which at least 60% w/w 1-octacosanol.

2. The composition of claim 1, wherein said monacolin K and said mixture of neoeriocitrin, naringin and neohesperidin are in a weight ratio of 1:10 to 1:18, preferably of 1:12 to 1:15, more preferably of 1:12,5 at 1:14.

3. The composition of claim 1 or 2, wherein said polycosanols and said mixture of neoeriocitrin, naringin and neohesperidin are in a weight ratio of 1:3 to 1:10, preferably of 1:6 to 1:7.

4. The composition of any one of claims 1-3, wherein said mixture of neoeriocitrin, naringin and neohesperidin is in the form of a Bergamot extract.

5. The composition of any one of claims 1-4, wherein said monacolin K is in the form of powder of red yeast rice titrated in monacolin K, preferably titrated to 1 to 5% monacolin K.

6. The composition of any one of claims 1-5, further comprising coenzyme Q10.

7. The composition of claim 6, wherein the only active ingredients present are the mixture of neoeriocitrin, naringin and neohesperidin, monacolin K, policosanols and coenzyme Q10.

8. The composition of any one of claims 1-7, comprising 5-20% by weight of a mixture of neoeriocitrin, naringin and neohesperidin, 0.4-2% by weight of monacolin K, 0.5-2% by weight of policosanols, on total composition weight.

9. The composition of any one of claims 1-8, in the form of a unit dose comprising 15-240 mg of said mixture of neoeriocitrin, naringin and neohesperidin, 2-12 mg of monacolin K, 3.5 to 25 mg of policosanols.

10. The composition of claim 9, in the form of a unit dose comprising 50-150 mg of said mixture of neoeriocitrin, naringin and neohesperidin, 3.5 to 10.5 mg of monacolin K, 6-20 mg of policosanols.

11. Dietary supplement comprising the composition of any one of claims 1-10 and suitable food excipients.

12. The dietary supplement of claim 11, comprising 62-72 mg of said mixture of neoeriocitrin, naringin and neohesperidin, in the form of a Bergamot extract, 4.8-5.5 mg of monacolin K, in the form of powder of red yeast rice titrated to 3% monacolin K, 9-11 mg of policosanols, 1-4 mg of Coenzyme Q10, and 250-400 mg of food excipients.

13. A pharmaceutical formulation comprising the composition of any one of claims 1-10 and pharmaceutically acceptable excipients.

14. The composition of any one of claims 1-10 for use in the treatment of hyperlipidemia.

15. The composition of any one of claims 1-10 for use in the treatment of UV radiation skin protection.

**Patentansprüche**

1. Zusammensetzung, Monacolin K, Policosanole und eine Mischung aus Neoeriocitrin, Naringin und Neohesperidin umfassend, wobei das Monacolin K und die Mischung aus Neoeriocitrin, Naringin und Neohesperidin in einem Gewichtsverhältnis von 1:5 bis 1:20 vorliegen und die Policosanole in Form einer Mischung vorliegen, die mindestens 98 Gew.-% C24-C40-Fettalkohole umfasst, von denen mindestens 60 Gew.-% 1-Octacosanol sind.

2. Zusammensetzung nach Anspruch 1, wobei das Monacolin K und die Mischung aus Neoeriocitrin, Naringin und Neohesperidin in einem Gewichtsverhältnis von 1:10 bis 1:18, vorzugsweise von 1:12 bis 1:15, bevorzugter von 1:12,5 bis 1:14 vorliegen.

3. Zusammensetzung nach Anspruch 1 oder 2, wobei die Policosanole und die Mischung aus Neoeriocitrin, Naringin und Neohesperidin in einem Gewichtsverhältnis von 1:3 bis 1:10, vorzugsweise von 1:6 bis 1:7 vorliegen.

4. Zusammensetzung nach einem der Ansprüche 1 bis 3, wobei die Mischung aus Neoeriocitrin, Naringin und Neohesperidin in Form eines Bergamotteextrakts vorliegen.

5. Zusammensetzung nach einem der Ansprüche 1 bis 4, wobei das Monacolin K in Form von Pulver aus Rotschimmelreis vorliegt, der in Monacolin K titriert wird, vorzugsweise auf 1 bis 5 % Monacolin K titriert wurde.

6. Zusammensetzung nach einem der Ansprüche 1 bis 5, ferner das Koenzym Q10 umfassend.

7. Zusammensetzung nach Anspruch 6, wobei die Mischung aus Neoeriocitrin, Naringin und Neohesperidin, das Monacolin K, die Policosanole und das Koenzym Q10 die einzigen vorhandenen Wirkstoffe sind.

8. Zusammensetzung nach einem der Ansprüche 1 bis 7, 5-20 Gew. % einer Mischung aus Neoeriocitrin, Naringin und Neohesperidin, 0,4-2 Gew.-% Monacolin K, 0,5-2 Gew.-% Policosanole auf das Gesamtgewicht der Zusammensetzung umfassend.

9. Zusammensetzung nach einem der Ansprüche 1 bis 8 in der Form einer Dosiseinheit, 15-240 mg der Mischung aus Neoeriocitrin, Naringin und Neohesperidin, 2-12 mg Monacolin K, 3,5 bis 25 mg Policosanole umfassend.

10. Zusammensetzung nach Anspruch 9 in der Form einer Dosiseinheit, 50-150 mg der Mischung aus Neoeriocitrin, Naringin und Neohesperidin, 3,5 bis 10,5 mg Monacolin K, 6 bis 20 mg Policosanole umfassend.

11. Nahrungsergänzungsmittel, die Zusammensetzung nach einem der Ansprüche 1 bis 10 und geeignete Lebensmittel-Hilfsstoffe umfassend.

12. Nahrungsergänzungsmittel nach Anspruch 11, 62-72 mg der Mischung aus Neoeriocitrin, Naringin und Neohesperidin in der Form eines Bergamotteextrakts, 4,8-5,5 mg Monacolin K in der Form von Pulver aus Rotschimmelreis, titriert auf 3 % Monacolin K, 9-11 mg Policosanole, 1-4 mg Koenzym Q10 und 250-400 mg Lebensmittel-Hilfsstoffe umfassend.

13. Pharmazeutische Formulierung, die Zusammensetzung nach einem der Ansprüche 1 bis 10 und pharmazeutisch annehmbare Hilfsstoffe umfassend.

14. Zusammensetzung nach einem der Ansprüche 1 bis 10 zur Verwendung bei der Behandlung von Hyperlipidämie.

15. Zusammensetzung nach einem der Ansprüche 1 bis 10 zur Verwendung bei der Behandlung von Haut-UV-Strahlungsschutz.

**Revendications**

1. Composition comprenant de la monacoline K, des policosanols et un mélange de néoériocitrine, de naringine et de néohespéridine, dans laquelle ladite monacoline K et ledit mélange de néoériocitrine, de naringine et de néohespéridine sont dans un rapport de poids de 1:5 à 1:20 et lesdits policosanols sont sous la forme d'un mélange comprenant au moins 98 % p/p d'alcools gras en C24 à C40, dont au moins 60 % p/p de 1-octacosanol.

2. Composition selon la revendication 1, dans laquelle ladite monacoline K et ledit mélange de néoériocitrine, de naringine et de néohespéridine sont dans un rapport de poids de 1:10 à 1:18, de préférence de 1:12 à 1:15, plus préférentiellement de 1:12,5 à 1:14.

3. Composition selon la revendication 1 ou 2, dans laquelle lesdits policosanols et ledit mélange de néoériocitrine, de naringine et de néohespéridine sont dans un rapport de poids de 1:3 à 1:10, de préférence de 1:6 à 1:7.

4. Composition selon l'une quelconque des revendications 1 à 3, dans laquelle ledit mélange de néoériocitrine, de naringine et de néohespéridine est sous la forme d'un extrait de Bergamote.

5. Composition selon l'une quelconque des revendications 1 à 4, dans laquelle ladite monacoline K est sous la forme de poudre de levure de riz rouge titrée en monacoline K, de préférence titrée à 1 à 5 % de monacoline K.

6. Composition selon l'une quelconque des revendications 1 à 5, comprenant en outre du coenzyme Q10.

7. Composition selon la revendication 6, dans laquelle les seuls ingrédients actifs présents sont le mélange de néoériocitrine, de naringine et de néohespéridine, la monacoline K, les policosanols et le coenzyme Q10.

8. Composition selon l'une quelconque des revendications 1 à 7, comprenant de 5 à 20 % en poids d'un mélange de néoériocitrine, de naringine et de néohespéridine, de 0,4 à 2 % en poids de monacoline K, de 0,5 à 2 % en poids de policosanols, sur le poids total de la composition.

9. Composition selon l'une quelconque des revendications 1 à 8, sous la forme d'une dose unitaire comprenant de 15 à 240 mg dudit mélange de néoériocitrine, de naringine et de néohespéridine, de 2 à 12 mg de monacoline K, de 3,5 à 25 mg de policosanols.

10. Composition selon la revendication 9, sous la forme d'une dose unitaire comprenant de 50 à 150 mg dudit mélange de néoériocitrine, de naringine et de néohespéridine, de 3,5 à 10,5 mg de monacoline K, de 6 à 20 mg de policosanols.

11. Complément alimentaire comprenant la composition selon l'une quelconque des revendications 1 à 10 et des excipients alimentaires appropriés.

12. Complément alimentaire selon la revendication 11, comprenant de 62 à 72 mg dudit mélange de néoériocitrine, de naringine et de néohespéridine, sous la forme d'un extrait de Bergamote, de 4,8 à 5,5 mg de monacoline K, sous la forme de poudre de levure de riz rouge titrée à 3 % de monacoline K, de 9 à 11 mg de policosanols, de 1 à 4 mg de coenzyme Q10 et de 250 à 400 mg d'excipients alimentaires.

13. Formulation pharmaceutique comprenant la composition selon l'une quelconque des revendications 1 à 10 et des excipients pharmaceutiquement acceptables.

14. Composition selon l'une quelconque des revendications 1 à 10 destinée à être utilisée dans le traitement de l'hyperlipidémie.

15. Composition selon l'une quelconque des revendications 1 à 10 destinée à être utilisée dans le traitement de la protection de la peau contre le rayonnement UV.

Figure 1

**EP 2 719 287 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006037725 A1